# EUROPEAN PATENT APPLICATION

(11) **EP 4 671 762 A1**
(43) Date of publication of application: **31.12.2025**
(21) Application number: 24760630.4
(22) Date of filing: 26.02.2024
(51) Int. Cl.: G01N 33/543, G01N 33/58, G01N 33/533

(54) **MULTIPLE DIAGNOSTIC KIT COMPRISING BIOCOMPATIBLE QUANTUM DOT-POLYMER COMPOSITE**

(30) Priority: 24.02.2023 KR 20230025292
(71) Applicant: Ulsan National Institute of Science and Technology (UNIST), Eonyang-eup Ulju-gun Ulsan 44919 (KR); Uniqdot, Ulju-gun, Ulsan 44919 (KR)
(72) Inventor: PARK, Jongnam, Ulju-gun, Ulsan 44919 (KR); PARK, Jungsik, Ulju-gun, Ulsan 44919 (KR)
(74) Representative: Beck & Rössig European Patent Attorneys
(86) International application number: PCT/KR2024/002456
(87) International publication number: WO 2024/177442

(57) **Abstract**

The present invention relates to a kit for multiple detection of target specimens, comprising a plurality of conjugates for immune detection, the kit comprising a quantum dot-polymer composite, and an antibody conjugated to the surface of the quantum dot-polymer composite and specifically binding to a target specimen. Specifically, in the kit for multiple detection of target specimens, the quantum dot-polymer composite comprises three-dimensional self-assembled quantum beads composed of one or more quantum dots of an indium phosphide core-shell structure, surface-modified ligands placed on the surfaces of the quantum dots and one or more polymer ligands, the hydrodynamic diameter of the quantum beads is 270-300 nm, and the plurality of conjugates for immune detection have emission wavelengths that vary according to the type of target specimen.

## Description

### [Technical Field]

The present invention relates to a multiple diagnostic kit including a biocompatible quantum dot-polymer composite.

### [Background Art]

Semiconductor nanoparticles that exhibit optical properties have great potential for applications in bioimaging. Since quantum dots (QDs) exhibit fluorescence with a high quantum yield and their band gap may be tuned by adjusting their size, they have an advantage in that the wavelength band of light emitted can be easily controlled. Since these optical properties show very bright imaging and are stable, QDs are expected to be much more effective materials than previously used dyes or protein fluorophores for cell imaging.

Gold nanoparticles have been used in the related art to diagnose diseases, but since the color change of gold nanoparticles is not large, diagnosis through the color of gold nanoparticles has limitations in increasing sensitivity. Various studies have been continuously conducted to replace gold nanoparticles with semiconductor nanoparticles, and many studies have reported the use of CdSe-based toxic QDs as fluorescent materials to improve the diagnostic sensitivity of in vitro diagnostic kits. However, since in vitro diagnostic kits require direct handling by human hands, commercialization has been difficult in cases where toxicity issues have not been resolved.

Meanwhile, as a kit for detecting a target material, a kit in the form of a lateral flow assay (LFA) strip is widely used. The lateral flow assay strip has a sample pad, a conjugate pad and a detection unit arranged in sequence in the longitudinal direction. A biological sample is supplied to the sample pad from the outside, and the supplied biological sample flows sequentially in the longitudinal direction to the conjugate pad, the detection portion, and the adsorption pad by capillary action. The conjugate pad is coated with a material capable of binding to a target analyte (such as an antigen) in a biological sample, and an immune complex is formed through an antigen-antibody reaction. In the detection unit, a detection reagent is applied to bind to a target analyte in a biological sample, and binds to the immune complex formed in the conjugate pad to form a detection conjugate. However, in existing diagnostic technologies using nanoparticles, methods have been proposed to increase the number of reaction areas, for example, conjugate pads and detection units, typically formed on a strip in order to simultaneously test a plurality of specific disease factors through a single test. However, such existing technologies increase the length of the strip, and accordingly cause a problem of increasing the test volume of a biological sample and reaction time, and cause a non-specific antigen-antibody reaction, which leads to concern about the accuracy of the test deteriorating.

Against this technical background, there is a need for the development of a multiple diagnostic kit using biocompatible semiconductor nanoparticles, for example, QDs.

### [Disclosure]

### [Technical Problem]

One aspect is to provide a kit for multiple detection of a target specimen including a plurality of conjugates for immunodetection, each conjugate comprises a quantum dot-polymer composite and an antibody specifically binding to the target specimen and having excellent biocompatibility and improved detection efficacy.

Another aspect is to provide a method for detecting a plurality of target specimens, the method including contacting a sample including any target specimen with the plurality of conjugates for immunodetection.

### [Technical Solution]

In one aspect, the present invention provides a kit for multiple detection of a target specimen including a plurality of conjugates for immunodetection, each conjugate comprises a quantum dot-polymer composite; and an antibody conjugated to the surface of the quantum dot-polymer composite and specifically binding to the target specimen,
wherein the quantum dot-polymer composite includes a quantum bead having a 3D self-assembly structure composed of one or more quantum dots having a core-shell structure and a surface-modified ligand placed on the surface of the quantum dot and one or more polymer ligands, the one or more quantum dots are dispersed in the quantum bead, the core includes indium phosphide (InP), the shell has a thickness of 3 nm to 9 nm, the quantum bead has a hydrodynamic diameter (HD) of 270 nm to 300 nm, and the plurality of composites for immunodetection have different emission wavelengths that vary according to the type of target specimen.

According to an exemplary embodiment, the target specimen may be one or more of an autoantibody, a ligand, a natural extract, a peptide, a protein, a metal ion, a synthetic drug, a natural drug, a metabolite, a genome, a virus and a virus-derived product, and a bacterium and a bacterium-derived product.

According to an exemplary embodiment, the kit may include a plurality of composites for immunodetection classified according to the type of target specimen, and each of the plurality of the classified composites for immunodetection may have a different size of antibodies and a different size of quantum dots in the quantum dot-polymer composite.

According to an exemplary embodiment, the plurality of composites for immunodetection may include at least two immune composites selected from a first composite for immunodetection with an emission wavelength in a range of 600 nm to 700 nm; a second composite for immunodetection with an emission wavelength in a range of 500 nm to 600 nm; and a third composite for immunodetection with an emission wavelength in a range of 400 nm to 500 nm.

For example, the mixing ratio of the first composite for immunodetection, the second composite for immunodetection, and the third composite for immunodetection may be in a range of 1:1:1 to 1:1.5:3.

According to an exemplary embodiment, the conjugate for immunodetection may be one in which 20 to 30 parts by weight of the antibody are conjugated to 100 parts by weight of the quantum dot-polymer composite.

According to an exemplary embodiment, the shell may have a thickness of about 3 nm to about 8 nm, about 3 nm to about 7 nm, about 3 nm to about 6 nm, about 3 nm to about 5 nm, or about 3 nm to about 4 nm.

According to an exemplary embodiment, the shell of the quantum dot may include one or more inorganic particles of zinc telluride (ZnTe), zinc sulfide (ZnS), zinc oxide (ZnO), zinc selenide (ZnSe), zinc selenium sulfide (ZnSeS), indium gallium nitride (InGaN), indium gallium phosphide (InGaP), indium zinc phosphide (InZnP), copper selenium telluride (CuSeTe), copper indium selenide (CuInSe₂), copper indium sulfide (CuInS₂), silver indium sulfide (AgInS₂), and tin telluride (SnTe).

For example, the shell of the quantum dot may include two or more different types of inorganic particles. For example, the shell of the quantum dot may include zinc sulfide (ZnS) and zinc selenide (ZnSe).

According to an exemplary embodiment, the shell of the quantum dot may include a first shell layer and a second shell layer, wherein the first shell layer may include a first inorganic particle and the second shell layer may include a second inorganic particle. The first inorganic particle and the second inorganic particle may be selected from the inorganic particles exemplified in the aforementioned shells.

For example, the first shell layer may be interposed between the core of the quantum dot and the second shell layer, and a band gap of the second inorganic particle may be larger than a band gap of the first inorganic particle. For example, the band gap of the first inorganic particle and the second inorganic particle may be larger than the band gap of InP. From such a band gap configuration, electrons and holes may be concentrated from the second inorganic particle through the first inorganic particle to the InP, and as a result, an effect of improving optical efficiency may be obtained.

According to an exemplary embodiment, the surface-modified ligand may include one or more thiol groups. For example, the surface-modified ligand may be a ligand derived from a linear, branched, or aromatic compound including one or more thiol groups as a functional group.

According to an exemplary embodiment, the surface-modified ligand may be a C1-C20 alkylthiol group including one or more thiol groups.

According to an exemplary embodiment, the polymer ligand may be selected from a random copolymer, a block copolymer, a graft copolymer, or a combination thereof.

According to an exemplary embodiment, the polymer ligand may include a hydrophobic portion and a hydrophilic portion.

For example, the hydrophobic portion of the polymer ligand may be aligned toward the center direction of the quantum bead, and the hydrophilic portion of the polymer ligand may be aligned toward the outside direction of the quantum bead.

For example, the hydrophilic portion of the polymer ligand may be exposed to the outside of the quantum bead.

According to an exemplary embodiment, the hydrophilic portion may be in the form of a needle or a brush.

According to an exemplary embodiment, the polymer ligand may include one or more of an amine group, an amide group, a hydroxy group, a maleimide group, a thiol group, and a carboxylic acid group at its end.

According to an exemplary embodiment, the polymer ligand may be one or more selected from polystyrene, polyolefin, polyvinyl acetal, polyester, polyketone, polyether, polyvinyl pyridine, polycarbonate, polydiene, polyacrylic acid, polyamide, and polyimide; random copolymers and block copolymers thereof; polymers including a poly(maleic anhydride) group; and graft copolymers consisting of a poly(maleic anhydride) group and an alkylamine.

According to an exemplary embodiment, when there are two or more polymer ligands, the two or more polymer ligands may be the same or different.

According to an exemplary embodiment, when there are two or more quantum dots, the two or more quantum dots may be the same or different.

For example, 50 to 50,000 quantum dots may be contained in the quantum bead.

According to an exemplary embodiment, the antibody that specifically binds to the target specimen may be conjugated to the surface of the quantum dot-polymer composite via an isopeptide bond between a first peptide tag and a second peptide tag.

For example, the first peptide tag may include a cysteine (Cys) in its terminal region, and the second peptide tag may include a molecule that binds to the Fc region of the antibody specifically binding to the target specimen in its terminal region.

According to an exemplary embodiment, the kit may be implemented in the form of a lateral flow assay strip.

For example, the lateral flow assay strip may include a conjugate pad including the plurality of conjugates for immunodetection; and a detection unit including a single test zone in which a plurality of detection antibodies, each specifically binding to each of a plurality of target specimens, are immobilized.

For example, the lateral flow assay strip may further include an absorbent sample pad, and the absorbent sample pad, the conjugate pad, and the detection unit are sequentially arranged from one end to the other end.

### [Advantageous Effects]

Since a kit for multiple detection according to one aspect includes a quantum dot-polymer composite having excellent photostability, high luminescence and quantum efficiency and the quantum dot-polymer composite has independent luminescence properties, a plurality of target specimens can be simultaneously detected.

The kit for multiple detection according to one aspect can allow a plurality of target specimens in a single test line to be detected at an effective level by including a plurality of quantum dot-polymer composites capable of exhibiting independent luminescence characteristics and high sensitivity, thereby contributing to the simultaneous diagnosis of multiple diseases.

### [Description of Drawings]

FIG. 1 is a schematic view for describing a lateral flow assay strip system.
FIG. 2 is a schematic view illustrating the structure of a lateral flow assay strip according to an exemplary embodiment.
FIG. 3 shows the results of analyzing the detection efficacy of a lateral flow assay strip to which quantum beads having a diameter of 240 nm according to an exemplary embodiment are applied, via fluorescence intensity.
FIG. 4 shows the results of analyzing the detection efficacy of a lateral flow assay strip to which quantum beads having a diameter of 270 nm according to an exemplary embodiment are applied, via fluorescence intensity.
FIG. 5 shows the results of analyzing the detection efficacy of a lateral flow assay strip to which quantum beads having a diameter of 300 nm according to an exemplary embodiment are applied, via fluorescence intensity.
FIG. 6 shows the results of analyzing the detection efficacy of a lateral flow assay strip to which quantum beads having a diameter of 370 nm according to an exemplary embodiment are applied, via fluorescence intensity.
FIG. 7 shows the results of analyzing the detection efficacy of a lateral flow assay strip to which the composite for immunodetection having various conjugation ratios between quantum beads and antibodies according to an exemplary embodiment is applied, via fluorescence intensity.
FIG. 8 shows the results of analyzing the detection efficacy of a lateral flow assay strip including a composite for immunodetection formed by conjugating 100 µg of quantum beads having a diameter of 270 nm and 27.8 µg of antibody according to an exemplary embodiment, via fluorescence intensity.
FIG. 9 shows the results of analyzing the detection sensitivity of a lateral flow assay strip to which a composite for immunodetection having red, green, or blue emission characteristics according to one embodiment is applied, via fluorescence intensity.
FIG. 10 shows the results of analyzing the multiple detection efficacy of a lateral flow assay strip in which a plurality of composites for immunodetection having red, green, or blue emission characteristics are mixed in various ratios according to an exemplary embodiment, via fluorescence intensity.

### [Modes of the Invention]

Hereinafter, several exemplary embodiments of the present invention will be described in detail with reference to the accompanying drawings such that a person with ordinary skill in the art to which the present invention pertains can easily carry out the present invention. The present invention can be implemented in various different forms, and is not limited to the exemplary embodiments described herein.

In order to clearly describe the present invention, parts that are not relevant to the description will be omitted, and the same reference numerals will be used to refer to the same or similar elements throughout the specification.

In addition, the size and thickness of each configuration shown in the drawings are arbitrarily shown for convenience of description, and the present invention is not necessarily limited to those shown in the drawings.

It should be noted that the terms used below are merely used to describe specific embodiments and are not intended to limit the present creative idea. Singular expressions include plural expressions unless the context clearly indicates otherwise. Hereinafter, it is to be understood that the terms "include" or "have" are intended to designate the existence of features, numbers, steps, actions, elements, parts, components, materials or combinations thereof described in the specification, but not to preclude the possibility of the presence or addition of one or more other features or numbers, steps, actions, elements, parts, components, materials or combinations thereof. The symbol "/" used below may be interpreted as either "and" or "or" depending on the context.

According to one aspect, provided is a kit for multiple detection of target specimens, including a plurality of conjugates for immunodetection, each conjugate comprises a quantum dot-polymer composite and an antibody conjugated to the surface of the quantum dot-polymer composite and specifically binding to a target specimen,
wherein the quantum dot-polymer composite includes three-dimensional self-assembled quantum beads composed of one or more quantum dots of a core-shell structure, surface-modified ligands placed on the surfaces of the quantum dots and one or more polymer ligands, the one or more quantum dots are dispersed in the quantum bead, the core includes indium phosphide (InP), the shell has a thickness of 3 nm to 9 nm, the hydrodynamic diameter (HD) of the quantum beads is 270 nm to 300 nm, and the plurality of composites for immunodetection have different emission wavelengths that vary according to the type of target specimen.

As used herein, the term "kit for multiple detection of target specimens" may be used interchangeably with the terms "multiple diagnostic kit," "multiple discrimination kit" and "multiple assay kit" depending on the function and use of the target specimens, and the kit for multiple detection of target specimens may be used and modified in form by adopting a technical configuration known in the art, as long as it includes the plurality of conjugates for immunodetection for detecting and labeling the target specimens as one element.

In a specific exemplary embodiment, the kit for multiple detection of a target specimen may be for simultaneously detecting a plurality of target specimens, simultaneously determining the presence or absence of a plurality of target specimens, or simultaneously diagnosing the presence or absence of a single disease/plural diseases originating from a plurality of target specimens. For this purpose, the kit may include a plurality of composites for immunodetection classified according to the type of target specimen, and each of the plurality of the classified composites for immunodetection may have a different size of antibodies and a different size of quantum dots in the quantum dot-polymer composite. For example, the plurality of composites for immunodetection may be classified into a plurality of groups depending on the type of target specimen, and each of the plurality of groups may be prepared such that the sizes of the antibody specifically binding to the target specimen and a quantum dot, that is, a core, in the quantum dot-polymer composite differ based on the target specimen. Here, the size of the quantum dots may be adjusted, for example, by changing the thermal reaction time between precursor materials for generating quantum dots during the quantum dot synthesis process, and any technology known for adjusting the size of the quantum dots can be extended and applied without limitation.

In a specific exemplary embodiment, each of the plurality of composites for immunodetection has a different size of quantum dot, that is, core, in the quantum dot-polymer composite, and may include a quantum dot-polymer composite with red, green, or blue emission characteristics. For example, the plurality of composites for immunodetection may include at least two immune composites selected from the group consisting of a first composite for immunodetection with an emission wavelength in a range of 600 nm to 700 nm, a second composite for immunodetection with an emission wavelength in a range of 500 nm to 600 nm, and a third composite for immunodetection with an emission wavelength in a range of 400 nm to 500 nm.

In a specific exemplary embodiment, the plurality of composites for immunodetection may include a first composite for immunodetection with an emission wavelength in a range of 600 nm to 700 nm and having red emission characteristics; a second composite for immunodetection with an emission wavelength in a range of 500 nm to 600 nm and having green emission characteristics; and/or a third composite for immunodetection with an emission wavelength in a range of 400 nm to 500 nm and having blue emission characteristics. Here, the plurality of composites for immunodetection may simultaneously detect a plurality of target specimens in a single test line as an effect derived from each of the independent emission characteristics. For example, red, green, or blue emission characteristics indicating the detection of one target specimen, yellow, purple, or blue-green emission characteristics indicating the detection of two target specimens, and white emission characteristics indicating the detection of three target specimens may be selectively confirmed in a single test line.

To secure the effectiveness of multiple detection of target specimens, the first composite for immunodetection may be conjugated to an antibody having the relatively lowest sensitivity, the second composite for immunodetection may be conjugated to an antibody having relatively medium sensitivity, and the third composite for immunodetection may be conjugated to an antibody having the relatively highest sensitivity. In addition, to secure the effectiveness of multiple detection of target specimens, the mixing ratio of the first composite for immunodetection, the second composite for immunodetection, and the third composite for immunodetection may range from 1:1:1 to 1:1.5:3 (weight ratio). For example, the mixing ratio may range from 1:1:1 to 1:1:1.5 (weight ratio), range from 1:1:1 to 1:1:2 (weight ratio), range from 1:1:1 to 1:1.2:1.8 (weight ratio), range from 1:1:1 to 1:1.2:2.4 (weight ratio), range from 1:1:1 to 1:1.4:2.1 (weight ratio), range from 1:1:1 to 1:1.4:2.8 (weight ratio), range from 1:1:1 to 1:1.5:2.3 (weight ratio), or range from 1:1:1 to 1:1.5:3 (weight ratio).

More specifically, the mixing ratio of the first composite for immunodetection and the second composite for immunodetection may range from 1:1 to 1:3, for example, 1:1 to 1:1.5; 1:1 to 1:2.0; 1:1 to 1:2.5; or 1:1 to 1:3 (weight ratio), or the mixing ratio of the first composite for immunodetection and the third composite for immunodetection may range from 1:1 to 1:3, for example, 1:1 to 1:1.5; 1:1 to 1:2.0; 1:1 to 1:2.5; or 1:1 to 1:3 (weight ratio), or the mixing ratio of the second composite for immunodetection and the third composite for immunodetection may be 1:1 to 1:2, for example, 1:1 to 1:1.5; or 1:1 to 1:2. When the above-described mixing ratios are exceeded, there is concern in that the effectiveness of multiple detection may deteriorate. Therefore, when multiple detection is performed by applying the two or three types of composites for immunodetection, the composites may be mixed in the above-described mixing ratio range.

As used herein, the term "target specimen" may refer to a molecule or other substances in a sample, which is or are to be detected. For example, the target specimens may include antigenic materials, ligands (mono- or polyepitopes), haptens, antibodies, and combinations thereof. In addition, the target specimens may include toxins, organic compounds, proteins, peptides, microorganisms, amino acids, nucleic acids, hormones, steroids, vitamins, drugs, drug intermediates or by-products, bacteria, virus particles, yeast, fungi, protozoa, and metabolites of the substances or antibodies thereto, but are not necessarily limited thereto. Preferably, as the target specimen, it is possible to exemplify, for example, ferritin; creatine kinase MB (CK-MB); digoxin; phenytoin; phenobarbital; carbamazepine; vancomycin; gentamicin; theophylline; valproic acid; quinidine; luteinizing hormone (LH); follicle-stimulating hormone (FSH); estradiol, progesterone; C-reactive protein (CRP); lipocalins; IgE antibodies; cytokines; vitamin B2 micro-globulin; glycated hemoglobin (Gly. Hb); cortisol; digitoxin; N-acetylprocainamide (NAPA); procainamide; antibodies to rubella, such as rubella-IgG and rubella IgM, for example, antibodies to toxoplasmosis, such as toxoplasmosis IgG (Toxo-IgG) and toxoplasmosis IgM; testosterone; salicylates; acetaminophen; hepatitis B virus surface antigens (HBsAg); antibodies to hepatitis B core antigen, for example, anti-hepatitis B core antigens IgG and IgM (anti-HBc); human immunodeficiency viruses 1 and 2 (HIV 1 and 2); human T-cell leukemia viruses 1 and 2 (HTLV); hepatitis B e antigen (HBeAg); antibodies to hepatitis B e antigen (anti-HBe); coronavirus; influenza virus; thyroid stimulating hormone (TSH); thyroxine (T4); total triiodothyronine (Total T3); free triiodothyronine (Free T3); carcinoembryonic antigen (CEA); lipoproteins, cholesterol, and triglycerides; and alpha fetoprotein (AFP).

As used herein, the term "conjugate for immunodetection" may broadly refer to a specific substance and a label conjugated thereto. In the present invention, the specific substance may refer to an antibody in a conjugate for immunodetection as a substance capable of detecting a specimen in a sample through specific binding, and the label may refer to a quantum dot-polymer composite in a conjugate for immunodetection. Here, "specific binding" refers to the specificity derived from an antibody, and may refer to preferential binding to a defined target specimen or target substance.

As used herein, the term "antibody" may refer to not only fully polyclonal (or polyclonal) or monoclonal (monoclonal) antibodies, but also fragments thereof (for example, Fab, Fab', F(ab')2, Fv), single chain (ScFv), diabodies, nanobodies, multispecific antibodies formed from antibody fragments, mutations thereof, fusion proteins containing antibody portions, and any other modified arrangement of an immunoglobulin molecule containing an antibody recognition site of the desired specificity. Antibodies may include antibodies that fall into any category, such as IgG, IgA, or IgM (or a subclass thereof), and antibodies that do not belong to a particular class.

In a specific exemplary embodiment, to secure the effectiveness of multiple detection of target specimens,
the conjugate for immunodetection may include 20 to 30 parts by weight of the antibody conjugated to 100 parts by weight of the quantum dot-polymer composite. For example, the conjugate for immunodetection may include 20 to 30 parts by weight, 22 to 30 parts by weight, 24 to 30 parts by weight, 26 to 30 parts by weight, 28 to 30 parts by weight, 20 to 28 parts by weight, 22 to 28 parts by weight, 24 to 28 parts by weight, 26 to 28 parts by weight, 20 to 26 parts by weight, 22 to 26 parts by weight, 24 to 26 parts by weight, 20 to 24 parts by weight, 22 to 24 parts by weight, or 22 to 24 parts by weight of the antibody conjugated to 100 parts by weight of the quantum dot-polymer conjugate.

As described above, gold nanoparticles, which have been mainly used as a material for in vitro diagnostic kits, have had a problem in that, due to a reduction in diagnostic sensitivity, it is difficult to diagnose diseases in the early stages. Thus, although attempts have been made to use various types of quantum dot composites as materials for diagnostic kits, there are limitations to their application to the human body due to the inherent properties of quantum dots, which are sensitive to moisture, and the toxicity of inorganic particles. In addition, in order to simultaneously test a plurality of specific disease factors in a single test, a method of increasing the number of reaction areas, for example, conjugate pads and detection units, typically formed on the strip has been proposed, but this increases the length of the strip, which in turn increases the amount of biological sample specimen and reaction time, and there are problems such as the occurrence of non-specific antigen-antibody reactions.

The quantum dot-polymer composite according to the present invention can be applied as a diagnostic kit for diagnosing human diseases using biocompatible quantum dots which include biocompatible indium phosphide (InP) as a core and do not include elements known to be toxic, such as cadmium (Cd) and lead (Pb), can exhibit excellent photostability and quantum efficiency by having a unique structure, and can exhibit an effect of maximizing emission intensity and antigen-antibody binding strength through this.

As a specific description of the structure of the quantum dot-polymer composite of the present invention, the quantum dot-polymer composite includes a quantum bead having a 3D self-assembly structure composed of one or more quantum dots and one or more polymer ligands, and in regard to the structure of the quantum dot-polymer composite, the disclosure of Korean Application No. 10-2022-0016432 is incorporated herein by reference.

Specifically, the quantum bead includes a quantum bead core part formed by the quantum dots and the lipophilic portion of the polymer ligand, and a hydrophilic portion of the polymer ligand to be described below.

For example, the quantum bead may be a core-shell structure including a quantum bead core part formed by the quantum dots and the lipophilic portion of the polymer ligand, and a shell part including the hydrophilic portion of the polymer ligand.

For example, the quantum dots use nanoparticles of indium phosphide (InP) as the core, and the shell may include one or more inorganic particles of zinc telluride (ZnTe), zinc sulfide (ZnS), zinc oxide (ZnO), zinc selenide (ZnSe), zinc selenium sulfide (ZnSeS), indium gallium nitride (InGaN), indium gallium phosphide (InGaP), indium zinc phosphide (InZnP), copper selenium telluride (CuSeTe), copper indium selenide (CuInSe₂), copper indium sulfide (CuInS₂), silver indium sulfide (AgInS₂), and tin telluride (SnTe). The quantum dots do not include elements known to be toxic, such as cadmium (Cd) and lead (Pb), making them biocompatible, and thus can be used as materials for a human disease diagnostic kit.

Indium phosphide (InP) inherently has a problem of easily deteriorating in external environments such as moisture and oxygen, and there is also a limitation that their optical characteristics deteriorate during the surface treatment process to compensate for the instability of the InP. Therefore, there are various restrictions on the use of indium phosphide-based quantum dots.

In an effort to compensate for the instability of InP, core-shell quantum dots, in which a shell is applied to the surface of an InP core particle, are known. In such InP quantum dots, the deterioration in InP caused by the shell is suppressed, but when the thickness of the shell is increased, it may cause a decrease in optical efficiency due to the generation of lattice defects, an increase in the number of defects due to mismatch in the interlayer crystal structure, or light scattering or light blocking effects caused by the crystals of the shell present in the light emission path of light converted and emitted from the core particle, and therefore it is very important to appropriately adjust the thickness of the shell in the preparation of quantum dots, and it is generally known that when the thickness of the shell increases, optical efficiency decreases.

In this regard, the shell thickness of typically available InP core-shell quantum dots is about 2 nm ± 0.5 nm, and InP quantum dots with this shell thickness are known to have optical efficiencies comparable to those of other quantum dots.

These typical InP quantum dots have problems in that defects caused by the inherent instability of the quantum dots during the process of forming quantum beads according to the present invention occur and the quantum dots are not fully dispersed in the beads due to insufficient interaction with the polymer ligands, resulting in a deterioration in the optical efficiency of the quantum bead. Therefore, in the process of exploring various methods to solve these problems, according to the present invention, it was found that when the thickness of the InP quantum dot shell is configured to be 3 nm to 9 nm and the surface of the quantum dot is modified with a surface-modified ligand, for example, one or more thiol-based compound-derived ligands including a thiol group, the indium phosphide-based quantum dots may be sufficiently included and dispersed in the quantum beads, resulting in sufficient emission efficiency for application to a conjugate for immunodetection and a diagnostic kit including the same.

According to an exemplary embodiment, the quantum bead includes one or more quantum dots, and in this case, when there are two or more quantum dots, the two or more quantum dots may be the same or different.

In addition, the polymer ligand may include a lipophilic portion that interacts with the quantum dot to form the central part of the quantum bead, and a hydrophilic portion that aids in aqueous dispersibility and is used to bind to antibodies that are used for application to a diagnostic kit.

In this case, the polymer ligand may be a random copolymer, a block copolymer, a graft copolymer, or a combination thereof. Here, the random copolymer is a copolymer in which two or more different types of units are randomly arranged. Furthermore, the block copolymer is a copolymer in which two or more different types of polymer chains are linked together through chemical bonds.

The block copolymer is in a form in which polymer chains to which hydrophilic units are bonded and polymer chains to which lipophilic units are bonded are linked, and the random copolymer is in a form in which hydrophilic units and lipophilic units are randomly arranged.

The quantum beads may be formed by hydrophobic interactions between the quantum dots and the lipophilic portion of the polymer ligand, and aqueous dispersion and binding strength with antibodies are secured by the hydrophilic portion of the polymer ligand extending to the surface of the core part of the quantum bead formed by the quantum dots and the lipophilic portion of the polymer ligand.

According to an exemplary embodiment, the hydrophobic portion of the polymer ligand may be aligned in the center direction of the quantum bead, and the hydrophilic portion of the polymer ligand may be aligned in the outside direction of the quantum bead.

According to an exemplary embodiment, the hydrophilic portion of the polymer ligand may be in the form of a needle or in the form of a brush.

Here, "form of a needle" refers to a form in which the hydrophilic portion of the polymer ligand is generally short and has a single functional group, and "form of a brush" refers to a form in which the hydrophilic portion of the polymer ligand is generally long and has several or more functional groups.

For example, the hydrophilic portion of the polymer ligand is not particularly limited, but may be in the form of a brush. When the hydrophilic portion of the polymer ligand is in the form of a brush as described above, compared to the form of a needle, when an antibody binds to the polymer ligand, as will be described below, the surface area for the hydrophilic portion of the polymer ligand binding to the antibody is increased, allowing a greater amount of antibody to bind.

For example, the polymer ligand is not particularly limited, but may be in the form of a brush in the case of a block copolymer, and may be in the form of a needle in the case of a random copolymer and a graft copolymer.

As the content of the antibody is increased, the binding form of the antibody in each quantum dot-polymer composite may be confirmed. In the case of a quantum dot-polymer composite including the hydrophilic portion of a polymer ligand in the form of a brush, even though the content of the antibody is increased, antibodies bind well to the surface of the hydrophilic portion of the polymer ligand because, as described above, the surface area for the hydrophilic portion of the polymer ligand binding to the antibody is large.

In contrast, in the case of a quantum dot-polymer composite including the hydrophilic portion of a polymer ligand in the form of a needle, when the content of the antibody is increased, the surface area for the hydrophilic portion of the polymer ligand binding to the antibody is insufficient, and the antibodies aggregate with each other rather than on the surface of the hydrophilic portion of the polymer ligand, and thus, the antibody binding efficiency may be slightly lower than that of the hydrophilic portion of a polymer ligand in the form of a brush.

Furthermore, when the quantum dot-polymer ligand composite of the present invention is applied to the LFA system, it can be confirmed that antigens bind to antibodies more effectively when the hydrophilic portion of the polymer ligand is in the form of a brush than when the hydrophilic portion of the polymer ligand is in the form of a needle.

For example, the polymer ligand may include one or more of an amine group, an amide group, a hydroxy group, a maleimide group, a thiol group, and a carboxylic acid group at its end.

For example, the polymer ligand may be one or more selected from polystyrene, polyolefin, polyvinyl acetal, polyester, polyketone, polyether, polyvinyl pyridine, polycarbonate, polydiene, polyacrylic acid, polyamide, and polyimide; random copolymers and block copolymers thereof; polymers including a polymaleic anhydride group; and graft copolymers consisting of a polymaleic anhydride group and an alkylamine.

For example, the polymer including a polymaleic anhydride group may be one or more selected from block copolymers; polymers including a polymaleic anhydride group; and graft copolymers consisting of a polymaleic anhydride group and an alkylamine.

For example, the polymer including a polymaleic anhydride group may be represented by the following Chemical Formula 1, and the graft copolymer consisting of a polymaleic anhydride group and an alkylamine may be represented by the following Chemical Formula 1A or 1B:

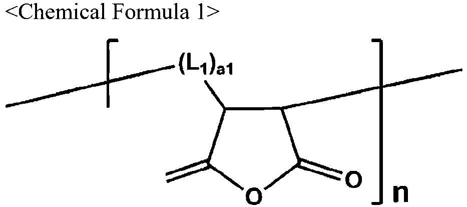

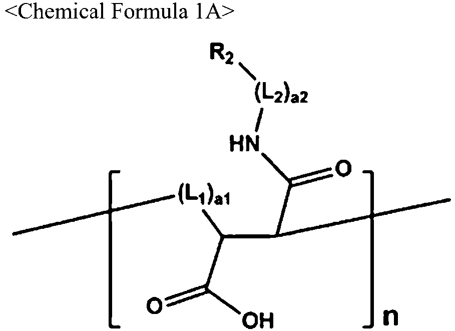

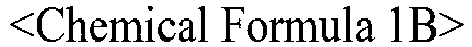

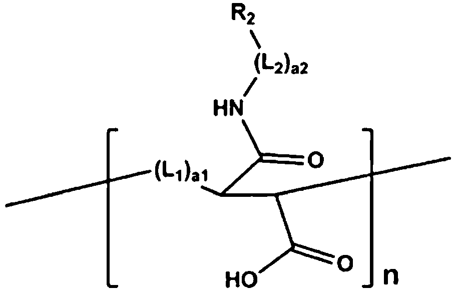

in Chemical Formulae 1, 1A and 1B,
L₁ and L₂ are each independently selected from a C₁-C₂₀ alkylene group; and a C₁-C₂₀ alkylene group substituted with one or more selected from deuterium, -F, -Cl, -Br, -I, a cyano group, a nitro group, a C₁-C₁₀ alkyl group, a C₆-C₂₀ aryl group, and a C₁-C₁₀ alkoxy group,
a1 is an integer selected from 1 to 5,
a2 is an integer selected from 0 to 5,
R₂ is selected from a C₁-C₂₀ alkyl group; and a C₁-C₂₀ alkyl group substituted with one or more selected from deuterium, -F, -Cl, -Br, -I, a cyano group, a nitro group, a C₁-C₁₀ alkyl group, a C₆-C₂₀ aryl group, and a C₁-C₁₀ alkoxy group, and
n is 1 or more and 1000 or less.

For example, in Chemical Formulae 1A and 1B,
L₁ and L₂ may each be independently selected from a methylene group, an ethylene group, a propylene group, an isopropylene group, a butylene group, an isobutylene group, a pentylene group, a hexylene group, a heptylene group, an octylene group, nonylene, and decylene; and a methylene group substituted with one or more selected from a methyl group, an ethyl group, a propyl group, a butyl group, a pentyl group, a hexyl group, a phenyl group, a methoxy group, and an ethoxy group, an ethylene group, a propylene group, an isopropylene group, a butylene group, an isobutylene group, a pentylene group, a hexylene group, a heptylene group, an octylene group, nonylene, and decylene.

For example, in Chemical Formulae 1A and 1B,
R₂ may be selected from a methyl group, an ethyl group, a propyl group, an isopropyl group, a butyl group, an isobutyl group, a pentyl group, a hexyl group, a heptyl group, an octyl group, a nonyl group, and a decyl group.

For example, the polymer ligand may be a random copolymer of an alkylene maleic anhydride monomer and an alkylamine monomer.

For example, the polymer ligand may be a graft copolymer of a poly alkylene maleic anhydride polymer and an alkylamine monomer.

For example, the polymer ligand may be a poly(isobutylene-alt-maleic anhydride)-hexadecylamine graft copolymer.

In this case, when there are two or more polymer ligands, the two or more polymer ligands may be the same or different.

According to an embodiment, the hydrodynamic diameter (HD) of the quantum bead may be from 50 nm to 1000 nm. For example, the hydrodynamic diameter of the quantum bead may be 270 nm to 300 nm, 275 nm to 300 nm, 280 nm to 300 nm, 285 nm to 300 nm, 290 nm to 300 nm, 295 nm to 300 nm, 270 nm to 295 nm, 275 nm to 295 nm, 280 nm to 295 nm, 285 nm to 295 nm, 290 nm to 295 nm, 270 nm to 290 nm, 275 nm to 290 nm, 280 nm to 290 nm, 285 nm to 290 nm, 270 nm to 285 nm, 275 nm to 285 nm, 280 nm to 285 nm, 270 nm to 280 nm, 275 nm to 280 nm, or 270 nm to 275 nm. Here, the hydrodynamic diameter of the quantum beads may be adjusted, for example, by changing the content of polymer ligands during the process of synthesizing quantum dots, and techniques known in the art for adjusting the hydrodynamic diameter of quantum beads can be extended and applied without limitation.

In this case, in situations outside of the above range, when the hydrodynamic diameter of the quantum bead is less than 270 nm, the emission intensity is so weak due to the small number of quantum dots included inside that there is a problem in that the detection sensitivity for a plurality of target specimens is weak. In contrast, when the hydrodynamic diameter of the quantum bead exceeds 300 nm, it is difficult to synthesize the quantum bead with a uniform size, and agglomeration occurs during the process of conjugating the antibody, causing a problem in that the composite for immunodetection gets stuck on the membrane and generates a background signal.

For example, the number of quantum dots forming the core part of the quantum bead may be appropriately adjusted according to the size of the quantum bead, and is not particularly limited, but may be, for example, 50,000 or less, for example, 50 to 50,000. For example, the number of quantum dots may be 40,000 or less, for example, 30,000 or less, for example, 20,000 or less, for example, 10,000 or less, for example, 5,000 or less, for example, 4,000 or less, for example, 3,000 or less, for example, 1,500 or less, for example, 50 to 1,500, for example, 50 to 1450, for example, 60 to 1,450, for example, 60 to 1,400, for example, 70 to 1,400, for example, 70 to 1,350, and for example, 80 to 1,350.

In this case, in situations outside of the above range, when the number of quantum dots forming the core part of the quantum bead exceeds 1,500, emission quenching between neighboring quantum dots may occur, and in contrast, when the number of quantum dots is less than 50, there is a problem of weak emission intensity.

The emission wavelength of the quantum bead is preferably 450 nm to 700 nm, but is not limited thereto.

In a specific exemplary embodiment, an antibody that specifically binds to the target specimen may be conjugated to the surface of the quantum dot-polymer composite via an isopeptide bond between a first peptide tag and a second peptide tag.

For example, the first peptide tag and the second peptide tag may be a pair of SpyTag and SpyCatcher; a pair of SnoopTag or SnoopTagJr and SnoopCatcher; a pair of RrgATag, RrgATag2 or DogTag and RrgACatcher, a pair of IsopepTag and Pilin-C; a pair of IsopepTag-N and Pilin-N; a pair of PsCsTag and PsCsCatcher; and a pair of SnoopTagJr and DogTag mediated by SnoopLigase, and preferably, the first peptide tag and the second peptide tag may be a pair of SpyTag and SpyCatcher.

Specifically, the first tag may be conjugated to the surface of the quantum dot-polymer composite, and the second tag may be conjugated to an antibody that specifically binds to the target specimen. For this purpose, the first tag may include at least one cysteine (Cys) in its terminal region (SC-Cys), and the second tag may include a molecule that binds to the FC region of an antibody that specifically binds to the target specimen (ST-Nb) in its terminal region. The molecule that binds to the Fc region of the antibody does not form a bond with the target specimen, but by forming a specific bond in the Fc region of an antibody that specifically binds to the target specimen, the molecule may confer functionality that exposes the Fab region of the antibody to the outside. As the molecule, for example, other antibodies or fragments thereof having the above-described specific binding may be applied, but it is not limited thereto.

Here, the terminal region of the first peptide tag is a region other than a region that forms a bond with the second tag, and may be conjugated to a surface-modified ligand in a quantum dot-polymer composite, specifically, form a disulfide bond with the thiol group of the surface-modified ligand. In addition, the terminal region of the second peptide tag is a region other than a region that forms a bond with the first tag, and may include a molecule that binds to the Fc region of an antibody not only to more densely conjugate an antibody with a Y-shaped structure to the surface of the quantum dot-polymer composite, but also to adjust the orientation of the antibody to be adjusted such that the Fab region is exposed to the outside. Such a conjugate structure between the quantum dot-polymer composite and the antibody may ultimately contribute to improving the detection sensitivity for the target specimen.

In a specific exemplary embodiment, a kit for detecting the target specimen is for detecting an antigen applicable to an antigen-antibody system, and may be an in vitro diagnostic kit based on the antigen. For example, the in vitro diagnostic kit may be for early diagnosis of various diseases (cancer, heart disease, COVID-19, and infectious diseases such as malaria). In addition, the kit 220 for detecting the target specimen may be implemented in the form of a lateral flow assay (LFA) strip.

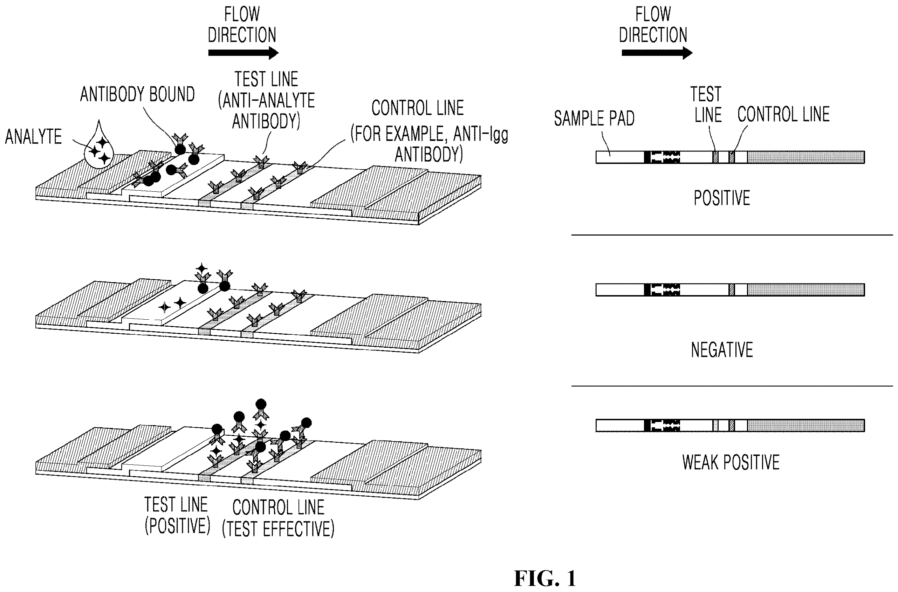

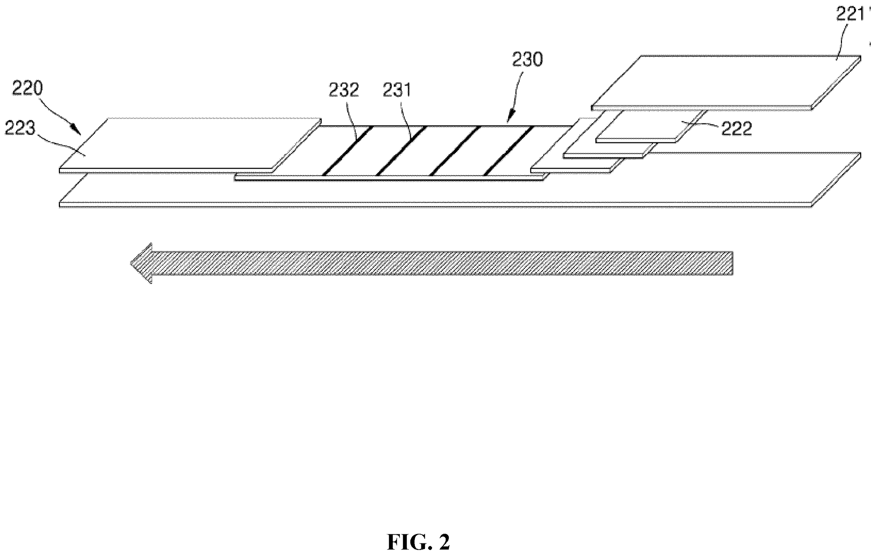

For example, referring to FIGS. 1 and 2, the lateral flow assay strip 220 may include a conjugate pad 222 including the plurality of conjugates for immunodetection; and a detection unit 230 including a single test zone 231 in which a plurality of detection antibodies, each specifically binding to each of a plurality of target specimens, are immobilized, and a control zone 232 in which control antibodies that do not specifically bind to the target specimen but that specifically bind to the detection antibodies that specifically bind to the target specimen in the conjugate for immunodetection are immobilized.

For example, referring to FIG. 2, the lateral flow assay strip 220 may include an absorbent sample pad 221, a conjugate pad 222, and a detection unit 230. The absorbent sample pad 221 may absorb a sample (test liquid), and the absorbent sample pad 221 may be prepared in a dry state such that a sample can be well absorbed. The conjugate pad 222 is located at the bottom or an adjacent surface of the absorbent sample pad 221, and may include a plurality of conjugates for immunodetection, which react with target specimens, as described above. The sample absorbed from the absorbent sample pad 221 is transferred to the conjugate pad 222, and when a plurality of target specimens are present in the sample, the reaction of each conjugate for immunodetection in the conjugate pad 222, specifically, an antigen-antibody reaction, results in the formation of an immune composite. The detection unit 230 may include a test zone or test line 231 coated with a material capable of binding to the formed immune composite, and a control zone or control line 232 coated with a raw material used for a control. For example, the control line 232 may include a visible band, and the test line 231 may be one that may be changed to a visible band by the reaction. The detection unit 230 may also be referred to as a membrane, and the material may be made of nitrocellulose, but other materials may be used, if needed.

For example, in the test line 231 of the detection unit 230, a detection antibody immobilized on the test line may form a bond with a [target specimen (antigen)-conjugate for immunodetection including a first antibody], a plurality of bonds may be present according to the combination of target specimens and the corresponding conjugates for immunodetection, and for purposes of the present invention, the test line may be a single zone. In addition, at the control line 231 of the detection unit 230, a control antibody immobilized against the control line may form a bond with the remaining conjugate for immunodetection, which has not formed an immune composite at the conjugate pad 222. Therefore, the above-described conjugate for an immune detection-based antigen-antibody reaction may not only confirm the presence or absence of the target specimen, but may also quantitatively assess the level of the target specimen, if needed.

For example, in the lateral flow assay strip 220, the absorbent sample pad 221, the conjugate pad 222, and the detection unit 230 may be sequentially arranged in a direction from one end to the other end. Specifically, the absorbent sample pad 221, the conjugate pad 222, and the detection unit 230 may be arranged such that, from one side, the absorbent sample pad 221, the conjugate pad 222, and the detection unit 230 are bound to overlap or be adjacent to each other.

For example, the lateral flow assay strip 220 may further include a hygroscopic pad 223 capable of absorbing a sample that has not reacted between the conjugate pad 222 and the detection unit 230. The hygroscopic pad 223 may absorb a sample that has not reacted between the conjugate pad 222 and the detection portion 230 while coming into contact with a zone adjacent to the control line provided in the detection unit 230. The hygroscopic pad 223 may be made of, but is not limited to, a cellulose material, and the hygroscopic pad 223 may be prepared in a dry state such that the sample can be well absorbed.

According to another aspect, provided is a method for detecting a plurality of target specimens, the method including contacting a sample including any target specimen with the plurality of conjugates for immunodetection.

Among terms or elements referred to in the method for detecting a target specimen, those referred to in the description of the kit for multiple detection of target specimens are as described above.

As used herein, the term "sample" is not particularly limited, so long as it can contain the target specimen to be detected. For example, the sample may be a biological sample, for example, a biological fluid or biological tissue. Examples of the biological fluid include urine, blood (whole blood), plasma, serum, saliva, semen, feces, sputum, cerebrospinal fluid, tears, mucus, amniotic fluid, and the like. The biological tissue is an aggregation of cells, and generally intracellular substances that form one of the structural substances of humans, animals, plants, bacteria, fungi, or viral structures, and specific types of assemblages, such as connective tissue, epithelial tissue, muscle tissue, nervous tissue, and the like may correspond to the biological tissue. Furthermore, examples of the biological tissue may also include organs, tumors, lymph nodes, arteries, and individual cell(s).

As used herein, the term "contact" refers to direct contact between two materials, but it can be broadly understood that any additional components may be present, as long as contact is made between the components and the liquid flow.

The present invention is described in more detail through the following synthesis examples and experimental examples. However, the synthesis examples are intended to illustrate the present invention and are not intended to limit the scope of the present invention.

### (Synthesis Examples)

### Synthesis Example 1: Synthesis of InP QD

0.8 mmol of InP, 1.6 ml of trioctylphosphine, 3.6 mmol of zinc (5.97 ml of a solution of zinc oleate dissolved in trioctylamine), and 0.5 mmol of selenium (0.25 ml of a solution of selenium dissolved in trioctylphosphine) were placed in a 3-neck flask and degassed using a vacuum pump.

Thereafter, after purging with argon, the flask was heated to 180°C under an argon gas atmosphere, and 0.15 ml of a mixed solution of hydrofluoric acid-trioctylamine with a volume ratio of 1:1 was injected to proceed with the reaction for 30 minutes.

The temperature was then raised to 340°C, 1.4 mmol of zinc (2.33 ml of a solution of zinc oleate dissolved in trioctylamine) was injected once, and 0.5 mmol of selenium (0.25 ml of a solution of selenium dissolved in trioctylphosphine) was injected twice to form a ZnSe shell, and in order to form the ZnS shell, the temperature was lowered to 320°C and 1.2 mmol of zinc (2.0 ml of a solution of zinc oleate dissolved in trioctylamine) and 0.25 mmol of sulfur (0.125 ml of a solution of sulfur dissolved in trioctylphosphine) were alternately injected twice and eight times, respectively.

Thereafter, 0.5 mmol of sulfur (0.25 ml of a solution of sulfur dissolved in trioctylphosphine) was injected once more and the reaction was maintained at 260°C for 1 hour, the temperature was lowered to 180°C and 0.15 ml of 1-dodecanethiol was injected to add the ligand to the surface of the quantum dots, and the synthesis was terminated.

The shell thickness was calculated by considering the diameter of the InP nanoparticles (3 nm) from the size of the quantum dot, and it was confirmed that the shell thickness was about 3.75 nm.

Thereafter, after the quantum dots (0.6 mg) synthesized above and a polystyrene-polyacrylic acid block copolymer (4 mg) (polymer ligand) were dispersed in tetrahydrofuran (1 ml), water (1 ml) was added while stirring, and then the resulting mixture was purified to prepare quantum beads.

### Synthesis Example 1-1. Synthesis of InP QDs with different hydrodynamic diameters

### (1) InP QDs having a diameter of 240 nm

After the quantum dots (0.6 mg) synthesized above and a polystyrene-polyacrylic acid block copolymer (4 mg) (polymer ligand) were dispersed in tetrahydrofuran (1 ml), water (1 ml) was added under stirring, and then the resulting mixture was purified to prepare quantum beads having a diameter of 240 nm.

### (2) InP QDs having a diameter of 270 nm

After the quantum dots (0.6 mg) synthesized above and a polystyrene-polyacrylic acid block copolymer (3.5 mg) (polymer ligand) were dispersed in tetrahydrofuran (1 ml), water (1 ml) was added under stirring, and then the resulting mixture was purified to prepare quantum beads having a diameter of 270 nm.

### (3) InP QDs having a diameter of 300 nm

After the quantum dots (0.6 mg) synthesized above and a polystyrene-polyacrylic acid block copolymer (4.0 mg) (polymer ligand) were dispersed in tetrahydrofuran (1 ml), water (1 ml) was added under stirring, and then the resulting mixture was purified to prepare quantum beads having a diameter of 300 nm.

### (4) InP QDs having a diameter of 370 nm

After the quantum dots (0.6 mg) synthesized above and a polystyrene-polyacrylic acid block copolymer (4.5 mg) (polymer ligand) were dispersed in tetrahydrofuran (1 ml), water (1 ml) was added under stirring, and then the resulting mixture was purified to prepare quantum beads having a diameter of 370 nm.

### Synthesis Example 1-2: Synthesis of InP QDs with different emission characteristics

### (1) InP QDs having red emission characteristics

0.8 mmol of InP, 1.6 ml of trioctylphosphine, 3.6 mmol of zinc (5.97 ml of a solution of zinc oleate dissolved in trioctylamine), and 0.5 mmol of selenium (0.25 ml of a solution of selenium dissolved in trioctylphosphine) were placed in a 3-neck flask and degassed using a vacuum pump. Thereafter, after purging with argon, the flask was heated to 180°C under an argon gas atmosphere, and 0.15 ml of a mixed solution of hydrofluoric acid-trioctylamine with a volume ratio of 1:1 was injected to proceed with the reaction for 30 minutes, thereby synthesizing an InP core having a diameter of 4 nm. Thereafter, the process of Synthesis Example 1 was performed to synthesize InP QDs having red emission characteristics.

### (2) InP QDs having green emission characteristics

0.8 mmol of InP, 1.6 ml of trioctylphosphine, 3.6 mmol of zinc (5.97 ml of a solution of zinc oleate dissolved in trioctylamine), and 0.5 mmol of selenium (0.25 ml of a solution of selenium dissolved in trioctylphosphine) were placed in a 3-neck flask and degassed using a vacuum pump. Thereafter, after purging with argon, the flask was heated to 180°C under an argon gas atmosphere, and 0.15 ml of a mixed solution of hydrofluoric acid-trioctylamine with a volume ratio of 1:1 was injected to proceed with the reaction for 20 minutes, thereby synthesizing an InP core having a diameter of 3 nm. Thereafter, the process of Synthesis Example 1 was performed to synthesize InP QDs having green emission characteristics.

### (3) InP QDs having blue emission characteristics

0.8 mmol of InP, 1.6 ml of trioctylphosphine, 3.6 mmol of zinc (5.97 ml of a solution of zinc oleate dissolved in trioctylamine), and 0.5 mmol of selenium (0.25 ml of a solution of selenium dissolved in trioctylphosphine) were placed in a 3-neck flask and degassed using a vacuum pump. Thereafter, after purging with argon, the flask was heated to 180°C under an argon gas atmosphere, and 0.15 ml of a mixed solution of hydrofluoric acid-trioctylamine with a volume ratio of 1:1 was injected to proceed with the reaction for 15 minutes, thereby synthesizing an InP core having a diameter of 2 nm. Thereafter, the process of Synthesis Example 1 was performed to synthesize InP QDs having blue emission characteristics.

### Synthesis Example 2. Manufacture of conjugate for immunodetection

Antibodies used for the detection of target substances were conjugated to the surface of the quantum bead by a covalent bond coupling method. Specifically, water-soluble quantum beads (0.1 mg), EDC (59.94 µg), and sulfo-NHS (135.80 µg) were mixed in 200 µL of MES buffer (0.01 M, pH 6.0), wherein the molar ratio of EDC and sulfo-NHS was adjusted to 1:2. Subsequently, the mixture was activated in a thermomixer (25°C, 300 rpm) for 30 minutes, and then the quantum beads were separated by centrifugation and redispersed in 200 µL of PBS (0.01 M, pH 8.0). Thereafter, antibodies (25 µg) were added to the quantum beads and mixed for 3 hours. The synthesized conjugate for immunodetection was purified by centrifugation (13,000 rpm, 10 minutes) and 200 µL of a blocking buffer (1x PBS containing 0.1% (w/v) Tween-20 and 3% (w/v) BSA) was added, and then reacted for 30 minutes to block further reaction on the quantum-bead surface. The reaction-blocked conjugate for immunodetection was centrifuged (13,000 rpm, 10 minutes), the supernatant was discarded, and then the pelleted conjugate for immunodetection was resuspended in 100 µL of the blocking buffer. In the present synthesis example, the antibodies used were anti-COVID-19 antibody (Fapon Biotech), anti-Influenza A antibody (Sino Biological, Inc.), and anti-Influenza B antibody (Sino Biological, Inc.).

Meanwhile, optionally, a conjugate for immunodetection in which the orientation of the antibody was adjusted to expose the Fab region to the outside was prepared by adopting the synthesis process of the conjugate for immunodetection described above, but adding the step of introducing Fc region-specifically binding nanobody (Nb). As the Fc region-specifically binding nanobody, a camelidae-derived single monomeric variable Ab domain designed to specifically capture/bind the Fc region of an IgG1 antibody was used. Specifically, the quantum beads (QBs) prepared from Synthesis Example 1, EDC (59.94 µg), sulfo-NHS (135.80 µg), and AEM (276.11 µg) were mixed in 200 µL of 1x PBS (0.01 M, pH 7.4). After 1 hour of activation, the mixture was centrifuged (13,000 rpm, 10 minutes) to remove residual reaction reagents. To perform the coating of nanobodies on the pelletized quantum beads, SC-Cys (SpyCatcher-Cys, 43.48 µg) and ST-Nb (SpyTag-Nb, 52.21 µg) were sequentially added. After reaction for 12 hours, the Nb-coated quantum beads were centrifuged (13,000 rpm, 10 minutes), washed, and then redispersed in 200 µL of 1x PBS (pH 7.4). Thereafter, a target antibody was added for final conjugation.

### 2-1. Manufacture of conjugates for immunodetection with different conjugation ratios of quantum beads and antibodies

Conjugates for immunodetection were prepared in the same manner as in Synthesis Example 2, but the content of the conjugated/added antibody was adjusted to each of 36 µg, 27 µg, 18 µg, or 9 µg to prepare conjugates for immunodetection with different antibody conjugation ratios.

### (Experimental Examples)

### Experimental Example 1. Evaluation of sensitivity according to size of quantum bead

In the present Experimental Example, a conjugate for immunodetection including quantum beads having different diameters in Synthesis Example 1-1 was used to prepare a lateral flow assay strip which detects SARS-CoV-2 NP as a target specimen. For this purpose, a membrane (Nupore Filtration Systems), a sample pad (Millipore), a conjugate pad (BOREDA BIOTECH CO., LTD.), an absorbent pad (BOREDA BIOTECH CO., LTD.), and a laminate card (BOREDA BIOTECH CO., LTD.) were each purchased. Thereafter, the membrane was attached to a 30 cm long laminate card, and a test line (concentration 1.5 mg/mL) and a control line (concentration 0.5 mg/mL) were formed on the membrane, and then the membrane was dried at 37°C for 2 hours. Thereafter, the dried membrane was immersed in a blocking buffer (1X PBS containing 2% BSA) for 20 minutes, and then the blocked membrane was immersed again in a washing solution (1X PBS containing 0.05% sodium dodecyl sulfate) for 30 minutes. The washed membrane was dried in an oven at 37°C for 2 hours, and then the sample pad, conjugation pad, and absorbent pad prepared in advance were attached to the membrane, and then each was cut into a size of 4 mm. Meanwhile, the conjugate pad was prepared by dispensing a mixture of 1.2 µL of the conjugate for immunodetection and 10.8 µL of a dilution buffer (1x PBS containing 0.1% Tween-20, 1% BSA, and 10% sucrose) onto the conjugate pad and then drying the conjugate pad in a vacuum oven at room temperature for 30 minutes.

Thereafter, 80 µL of a sample solution prepared by diluting the SARS-CoV-2 NP stock solution with 1X PBS was dropped onto the sample pad of the prepared lateral flow assay strip, and allowed to pass through the membrane by capillary action. The concentrations of SARS-CoV-2 NP in the sample solutions were adjusted to each of 200 ng/ml, 20 ng/ml, 2 ng/ml, 200 pg/ml, 100 pg/ml, 50 pg/ml, and 20 pg/ml. All the solutions were absorbed into the absorbent pad within 20 minutes, and the results were measured and recorded using a portable UV lamp (UVITec LF-206. LS) and a Samsung Galaxy S20 smartphone. Thereafter, quantitative analysis was performed by scanning the images and measuring the fluorescence intensity with an AXIO ZOOM stereo microscope (Zeiss).

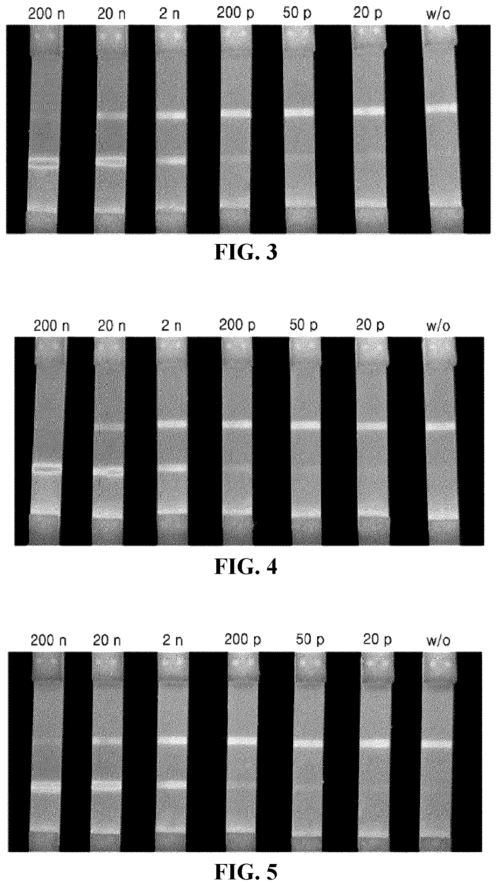

As a result, as shown in FIGS. 3 to 6, it was confirmed that as the quantum bead size increased, the number of antibodies to be conjugated increased, and thus effective detection efficacy could be exhibited even in samples including target specimens at the level of 50 pg/mL. However, it was confirmed that in a group using quantum beads with a size of 300 nm or more, not only did some agglomeration occur during the antibody conjugation process, but the detection efficacy was also reduced because the group got stuck on the membrane rather than the test line during the detection process and formed a background signal.

### Experimental Example 2. Sensitivity evaluation according to conjugation ratio between quantum beads and antibodies

In the present experiment, the sensitivity of a lateral flow assay strip according to an exemplary embodiment was evaluated using conjugates for immunodetection including quantum beads with a diameter of 270 nm and having different antibody conjugation ratios of Synthesis Example 2-1. Specifically, detection was performed on a sample solution including SARS-CoV-2 NP at a concentration of 20 ng/ml in the same manner as in Experimental Example 1. Thereafter, using a conjugate for immunodetection including quantum beads with a diameter of 270 nm conjugated with 27 µg of antibody, which exhibited optimal detection efficacy, the sensitivity in the lateral flow assay strip was reevaluated for sample solutions including SARS-CoV-2 NP at a concentration of 200 ng/ml, 20 ng/ml, 2 ng/ml, 200 pg/ml, 100 pg/ml, 50 pg/ml, and 20 pg/ml.

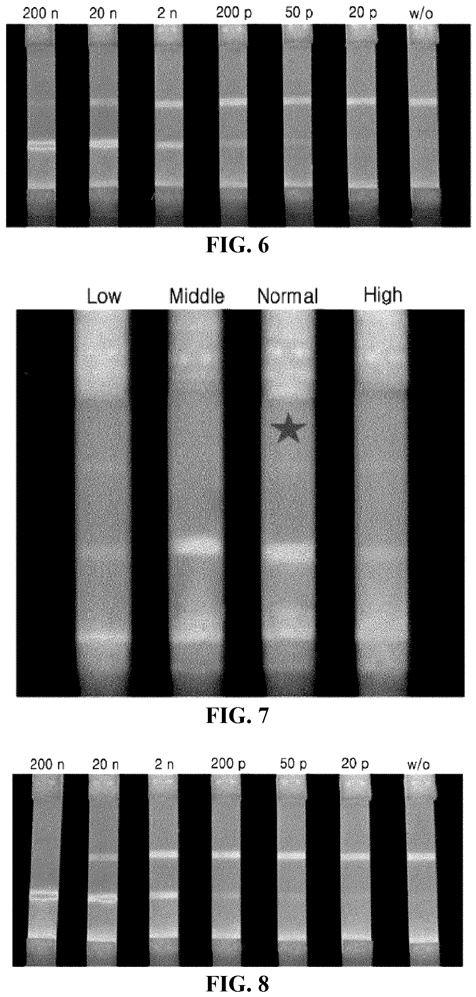

As a result, as shown in FIG. 7, it was confirmed that the conjugates for immunodetection conjugated with 18 µg or 27 µg of antibody (middle, normal) showed effective detection efficacy, whereas the composite for immunodetection conjugated with 9 µg of antibody (Low) had a problem with antigen detection due to a weak level of antibody for detection, and the composite for immunodetection conjugated with 36 µg of antibody (high) showed a sharp drop in antigen detection level due to the occurrence of agglomeration at the conjugate pad.

In addition, as shown in FIG. 8, it was confirmed that the conjugate for immunodetection including quantum beads with a diameter of 270 nm conjugated with 27 µg of antibody could exhibit effective detection efficacy even in a sample including a target specimen at a level of 50 pg/mL.

### Experimental Example 3. Evaluation of multiple detection efficacy by mixing plurality of quantum beads

In the present Experimental Example, the sensitivity of a lateral flow assay strip according to an exemplary embodiment was evaluated using composites for immunodetection of Synthesis Example 1-2 exhibiting different emission characteristics, that is, red, green, or blue, under the same antigen detection and antibody conditions. Specifically, solutions including three types of antigens (Influenza A, Influenza B, or SARS-CoV-2 antigens) at a concentration of 50 ng/mL, 20 ng/mL, 5 ng/mL, 2 ng/mL, 500 pg/mL, 200 pg/mL, 50 pg/mL, and 20 pg/mL, or including no antigen, were prepared separately, and then three types of solutions including antigens at the same concentration were diluted to prepare sample solutions at different concentrations. Thereafter, in the same manner as in Experimental Example 1, detection of each antigen was performed using composites for immunodetection showing red (conjugated to anti-Influenza A antibody), green (conjugated to anti-COVID-19 antibody), or blue (conjugated to anti-Influenza B antibody).

Subsequently, in reflection of the reactivity of antibodies against the target specimen (antigen) and the detection/reading sensitivity level according to the emission characteristics of the quantum beads, it was attempted to derive a mixing ratio between the composites for immunodetection, which enables effective multiple detection during the multiple detection of two or more types of composites for immunodetection mixed at various weight ratios in a sample solution including an antigen at a concentration of 20 ng/mL using a composite for immunodetection having red emission characteristics, which is conjugated to an anti-Influenza A antibody having the relatively lowest sensitivity, a composite for immunodetection having green emission characteristics, which is conjugated to an anti-COVID-19 antibody having relatively medium sensitivity, and a composite for immunodetection having blue emission characteristics, which is conjugated to an anti-Influenza B antibody having the relatively highest level of sensitivity.

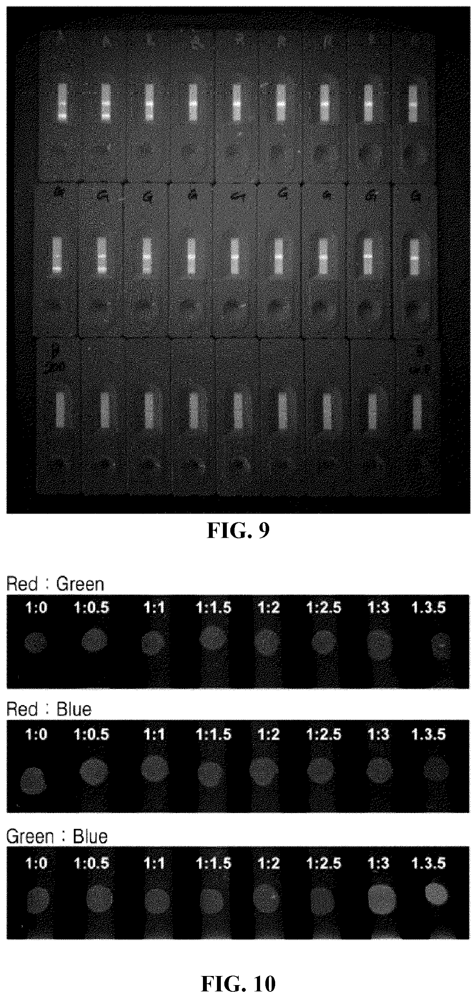

As a result, as shown in FIG. 9, it was confirmed that the composite for immunodetection having red emission characteristics was able to read the most sensitive results, and subsequently, the sensitivity relatively decreased in the order of green and blue.

Furthermore, as shown in FIG. 10, as a result of confirming the optimal ratio by mixing two or more colors, it was confirmed that effective multiple detection was possible when the composite for immunodetection having red emission characteristics and the composite for immunodetection having green emission characteristics were mixed in a weight ratio of 1:1 to 1:3, the composite for immunodetection having red emission characteristics and the composite for immunodetection having blue emission characteristics were mixed in a weight ratio of 1:1 to 1:3, and the composite for immunodetection having green emission characteristics and the composite for immunodetection having blue emission characteristics were mixed in a weight ratio of 1:1 to 1:2. That is, it was confirmed that effective multiple detection is possible when the composite for immunodetection having red emission characteristics, the composition for immunodetection having green emission characteristics, and the composite for immunodetection blue emission characteristics are mixed in a ratio of 1:1:1 to 1:1.5:3.

The above-described description of the present invention is provided for illustrative purposes, and a person skilled in the art to which the present invention pertains will understand that the present invention can be easily modified into other specific forms without changing the technical spirit or essential features of the present invention. Therefore, it should be understood that the above-described examples are only illustrative in all aspects and not restrictive.

## Claims

1. A kit for multiple detection of a target specimen comprising a plurality of conjugates for immunodetection, each conjugate comprises a quantum dot-polymer composite; and an antibody conjugated to a surface of the quantum dot-polymer composite and specifically binding to the target specimen,
wherein the quantum dot-polymer composite comprises a quantum bead having a 3D self-assembly structure composed of one or more quantum dots having a core-shell structure and a surface-modified ligand placed on the surface of the quantum dot and one or more polymer ligands,
the one or more quantum dots are dispersed in the quantum bead,
the core comprises indium phosphide (InP),
the shell has a thickness of 3 nm to 9 nm,
the quantum bead has a hydrodynamic diameter (HD) of 270 nm to 300 nm, and
the plurality of composites for immunodetection have emission wavelengths that vary according to the type of target specimen.

2. The kit of claim 1, wherein the target specimen is one or more of an autoantibody, a ligand, a natural extract, a peptide, a protein, a metal ion, a synthetic drug, a natural drug, a metabolite, a genome, a virus and a virus-derived product, and a bacterium and a bacterium-derived product.

3. The kit of claim 1, wherein the kit comprises a plurality of composites for immunodetection classified according to the type of target specimen, and each of the plurality of the classified composites for immunodetection has a different size of antibodies and a different size of quantum dots in the quantum dot-polymer composite.

4. The kit of claim 1, wherein the plurality of composites for immunodetection comprise at least two immune composites selected from the group consisting of a first composite for immunodetection with an emission wavelength in a range of 600 nm to 700 nm; a second composite for immunodetection with an emission wavelength in a range of 500 nm to 600 nm; and a third composite for immunodetection with an emission wavelength in a range of 400 nm to 500 nm.

5. The kit of claim 4, wherein a mixing ratio of the first composite for immunodetection, the second composite for immunodetection, and the third composite for immunodetection ranges from 1:1:1 to 1:1.5:3 (weight ratio).

6. The kit of claim 1, wherein the conjugate for immunodetection comprises 20 to 30 parts by weight of the antibody conjugated to 100 parts by weight of the quantum dot-polymer composite.

7. The kit of claim 1, wherein the shell comprises one or more inorganic particles of zinc telluride (ZnTe), zinc sulfide (ZnS), zinc oxide (ZnO), zinc selenide (ZnSe), zinc selenium sulfide (ZnSeS), indium gallium nitride (InGaN), indium gallium phosphide (InGaP), indium zinc phosphide (InZnP), copper selenium telluride (CuSeTe), copper indium selenide (CuInSe₂), copper indium sulfide (CuInS₂), silver indium sulfide (AgInS₂), and tin telluride (SnTe).

8. The kit of claim 1, wherein the surface-modified ligand comprises one or more thiol groups.

9. The kit of claim 1, wherein the polymer ligand is selected from a random copolymer, a block copolymer, a graft copolymer, or a combination thereof.

10. The kit of claim 1, wherein the polymer ligand comprises one or more of an amine group, an amide group, a hydroxy group, a maleimide group, a thiol group, and a carboxylic acid group at its end.

11. The kit of claim 1, wherein the polymer ligand is one or more selected from polystyrene, polyolefin, polyvinyl acetal, polyester, polyketone, polyether, polyvinyl pyridine, polycarbonate, polydiene, polyacrylic acid, polyamide, and polyimide; random copolymers and block copolymers thereof; polymers comprising a poly(maleic anhydride) group; and graft copolymers consisting of a poly(maleic anhydride) group and an alkylamine.

12. The kit of claim 1, wherein 50 to 50,000 quantum dots are contained in the quantum bead.

13. The kit of claim 1, wherein the antibody that specifically binds to the target specimen is conjugated to a surface of the quantum dot-polymer composite via an isopeptide bond between a first peptide tag and a second peptide tag.

14. The conjugates for immunodetection of claim 13, wherein the first peptide tag comprises a cysteine (Cys) in its terminal region, and
the second peptide tag comprises a molecule that binds to an FC region of the antibody specifically binding to the target specimen in its terminal region.

15. The kit of claim 1, wherein the kit is a lateral flow assay strip.

16. The kit of claim 15, wherein the lateral flow assay strip comprises a conjugate pad comprising the plurality of conjugates for immunodetection of claim 1; and a detection unit comprising a single test zone in which a plurality of detection antibodies, each specifically binding to each of a plurality of target specimens, are immobilized.

17. The kit of claim 16, wherein the lateral flow assay strip further comprises an absorbent sample pad, and
the absorbent sample pad, the conjugate pad, and the detection unit are sequentially arranged from one end to the other end.
